# EUROPEAN PATENT APPLICATION

(11) **EP 4 424 332 A1**
(43) Date of publication of application: **04.09.2024**
(21) Application number: 23159468.0
(22) Date of filing: 01.03.2023
(51) Int. Cl.: A61L 9/04, A61L 9/03, A61L 9/12

(54) **APPARATUS, SENSOR, SYSTEM AND METHOD FOR ADJUSTING A STABLE CONCENTRATION OF HYPOCHLOROUS ACID AND/OR ASSOCIATED FREE ACTIVE CHLORINE IN AMBIENT AIR FOR DISINFECTION**

(71) Applicant: oji Europe GmbH, 14641 Nauen (DE)
(72) Inventor: BOECKER, Dirk, 14641 Nauen (DE); BONE-WINKEL, Thomas, 14641 Nauen (DE)
(74) Representative: RGTH

(57) **Abstract**

To provide an apparatus for evaporating hypochlorous acid (HOCI) with which a stable and spatially homogeneous concentration of HOCI in ambient air, in particular in room air, can be adjusted and which prevents corrosive salts to be dispersed into the ambient air, an apparatus (100) for evaporating hypochlorous acid (HOCI), comprising a housing (10), a container (11) for an aqueous HOCI solution (17), and preferably a fan (12), is proposed, wherein the apparatus further comprises a substrate (13) with a high surface-to-volume ratio arranged to receive or to be wetted by an HOCI solution (17) from the container (11) during operation of the apparatus (100).

## Description

The present invention relates to an apparatus for evaporating hypochlorous acid (HOCI), comprising a housing, a container for an aqueous HOCI solution, and preferably a fan.

Furthermore, the present invention relates to a sensor for determining an HOCI concentration in ambient air, a system for adjusting a stable concentration of hypochlorous acid and/or associated free active chlorine in ambient air and a method for adjusting a stable concentration of hypochlorous acid and/or associated free active chlorine in ambient air for disinfection.

### Technological background

Hypochlorous acid (HOCI) is a weak acid that forms when chlorine dissolves in water, and itself partially dissociates, forming hypochlorite ClO⁻. The ratios of HOCl, ClO⁻ and Cl₂ in the resulting aqueous solution are pH dependent. Hypochlorous acid (HOCI) and ClO⁻ are oxidizers, and the primary disinfection agents of chlorine solutions denaturing and aggregating proteins and deactivating numerous organic compounds.

HOCI is an indigenous substance in mammals and is effective against a broad range of microorganisms. In aqueous solution, HOCI dissociates into H⁺ and ClO⁻. HOCI also destroys viruses, which can result in single as well as double stranded DNA breaks, rendering the nuclear acid useless and the virus harmless. An enveloped virus such as a corona virus will be primarily deactivated by the chlorine component (HOCI, ClO⁻and Cl₂) through reacting with and incapacitating their lipid shell/envelope. Microorganisms, in particular viruses and bacteria, are present everywhere in everyday life. Some of the viruses and microorganisms can be harmful to mammals in general and to humans in particular. With the Covid-19 epidemic caused by the SARS-CoV-2 virus the need for apparatuses, methods and systems for disinfecting air and surfaces has grown even more.

JP H11-169441 A discloses an indoor sterilization method for non-populated rooms capable of simultaneously sterilizing not only ceilings and walls but also the air by atomization of a disinfectant. The disinfectant is sterilized water containing hypochlorite with a pH between 3 and 7.5 and having a hypochlorous acid concentration of 10 to 200 ppm.

JP 2007-162997 A discloses a dry fog generator suitable for a non-populated waiting room or a patient's room of a hospital, for sterilizing or sanitizing floating fungus by nebulizing and emitting a chemical. The chemical is a hypochlorite solution, which is sprayed from a nozzle of a dry fog generator.

Prior art document US 2014/0119992 A1 discloses a disinfecting solution comprising a liquid having an HOCI concentration between 50 ppm and 4.000 ppm and a quantity of ethyl alcohol comprising between 0.05% and about 10% of the disinfectant solution by weight.

WO 2011/085470 A1 provides a delivery mechanism for dispersing a pathogen disinfectant liquid into the air or onto surfaces. The apparatus includes a container comprising a sunlight-resistant compartment containing a pathogen disinfectant liquid, a propellant, and an actuator coupled to a valve for opening and closing the valve, wherein opening of the valve causes the propellant to move at least a portion of the pathogen disinfectant liquid through an orifice in the container.

Prior art document CN 111481723 A discloses a method for disinfecting air in gathering places by using a weak hypochlorous acid disinfectant wherein the weak hypochlorous acid disinfectant has a pH of 5.0 to 6.5 and contains 10 ppm to 500 ppm hypochlorous acid and 10 ppm to 9.000 ppm sodium chloride. A cold fogging machine using high frequency ultrasonic vibrations between 5 kHz to 1.7 MHz disperses the weak hypochlorous acid disinfectant into the air at a rate of 0.5 to 1.5 l/hour.

Known apparatus for dispersing hypochlorous acid are based on the principle of spraying small droplets of an aqueous HOCI solution. This has two effects. First, because of the still considerable size of the droplets, these droplets will sink down to the floor by gravity and thus constantly be removed from the air. Second, the mean distance between the hypochlorous acid disinfectant droplets is much larger than the size of the aerosolized pathogen by at least one order of magnitude. This large mean distance reduces the probability that any passing aerosolized pathogen in the air comes into contact with the hypochlorous acid disinfectant droplets to practically zero. Third, the disinfectant droplets sprayed into the air contain additional ingredients such as salts and HOCI derivatives. These ingredients are partly harmful to health and are also corrosive.

### Disclosure of the invention: problem, solution, advantages

It is an object of the present invention to provide an apparatus for evaporating hypochlorous acid (HOCI) with which a stable and spatially homogeneous concentration of HOCI in ambient air, in particular in room air, can be adjusted and which prevents corrosive salts to be dispersed into the ambient air.

Furthermore, it is an object of the present invention to provide a sensor, a system and a method for adjusting a stable concentration of hypochlorous acid and/or associated free active chlorine in ambient air for disinfection.

To solve the object, the present invention proposes an apparatus for evaporating hypochlorous acid (HOCI), comprising a housing, a container for an HOCI solution, and preferably a fan, wherein the apparatus further comprises a substrate with a high surface-area-to-volume ratio arranged to receive or to be wetted by an HOCI solution from the container during operation of the apparatus.

The fan may be disposed inside or outside of the housing. If the apparatus is positioned in or configured for being positioned in an air duct of, for example, a ventilation system or an HVAC-system, the fan may be provided by the ventilation system or HVAC-system. The fan may also be referred to as a ventilator.

Thus, during operation of the apparatus an aqueous HOCI solution from the container is fed to the substrate having a high surface-area-to-volume ratio such that the substrate receives the HOCI solution and/or is wetted by the HOCI solution. HOCI from the HOCI solution received by or having wetted the substrate evaporates from the substrate and can be dispensed into the ambient air, in particular in the room air, for disinfection purposes.

Due to the high surface-area-to-volume ratio of the substrate, HOCI easily evaporates and can be dispensed into the ambient air in its gaseous form, so that salts and other, possibly corrosive, ingredients remain in the HOCI solution on the substrate without entering the ambient air to be disinfected.

Furthermore, because of the high surface-area-to-volume ratio of the substrate the higher partial vapor pressure of HOCI compared to the partial vapor pressure of water favors the evaporation of HOCI over the evaporation of water so that only a small amount of water compared to the HOCI evaporates and enters the ambient air. HOCI cannot be heated as it would lose its characteristics for air and surface disinfection. Thus, by using a substrate with a high surface-area-to-volume ratio, HOCI can be evaporated without heating solely by taking advantage of the higher partial vapor pressure of HOCI compared to the partial vapor pressure of water.

Preferably, the concentration of the HOCI in the aqueous HOCI solution is adjusted to be so high that the evaporation of the HOCI is high compared to the evaporation of the water due to its respective higher vapor pressures. In this way non-intended humidification effects can be minimized.

Still further preferably the substrate is a woven or non-woven fabric or a mesh or an open foam material, wherein the woven or non-woven fabric or mesh or open foam material is particularly preferably resistant to HOCI.

Preferably the substrate is a non-woven fabric without or with pores.

Also preferably, the substrate may be a mesh, further preferably a membrane filter.

The woven or non-woven fabric or the mesh or the membrane filter or the open foam material may have a pore size between 0.005 µm to 12 µm, further preferably between 0.1 µm to 5 µm, still further preferably between 0.2 µm and 0.5 µm. The woven fabric or the mesh may have a woven structure where the openings or spaces between the individual structural elements of the woven fabric or mesh have a spatial distance between 50 µm and 500 µm.

The fabric or the mesh and/or membrane filter and/or the open foam material may comprise polyvinylidene fluoride, polyethersulfone, polycarbonate, polytetrafluoroethylene, silver or polyvinyl chloride.

A woven or non-woven fabric or mesh or open foam material is particularly suited to receive the aqueous HOCI solution and/or to be wetted by the aqueous HOCI solution.

In a preferred embodiment the fan is arranged and configured to generate an airflow for transporting HOCI evaporated from the aqueous HOCI solution out of the apparatus, wherein said aqueous HOCI solution has been received by or has wetted the substrate.

In other words, HOCI evaporates from the HOCI solution that has been received by or has wetted the substrate and the evaporated HOCI in the gaseous phase is transported out of the apparatus into the ambient air, preferably using an air guide or tubing system, for example an air duct, to lead the HOCI enriched air to a desired area, by an airflow generated by the fan.

In case the apparatus is positioned in or configured for being positioned in an air duct of, for example, a ventilation system or an HVAC-system, the fan may be provided by the ventilation system or HVAC-system.

Further preferably the substrate and fan are arranged such that the airflow generated by the fan passes through the substrate. The substrate is preferably configured in a way that the pressure drop caused by the HOCI evaporation unit barrier is minimal.

Thus, the substrate is preferably air-permeable. The airflow generated by the fan passes through the substrate and transports the evaporated HOCI out of the apparatus.

It is also possible that the substrate and fan are arranged such that the airflow generated by the fan passes along, preferably over the surface of, the substrate.

Preferably, the substrate receives or is wetted by the HOCI solution by the HOCI solution running down the substrate following gravity or by the HOCI solution being absorbed or adsorbed by a capillary effect, and/or a fluid guiding means is provided and arranged to feed HOCI solution from the container to a top end and/or to a bottom end of the substrate.

Thus, the HOCI solution may be fed to the substrate at a top end of the substrate so that it flows down the substrate by gravity. In this case the substrate with a high surface-area-to-volume ratio receives, absorbs, adsorbs or is wetted by the HOCI solution flowing down the substrate. By constantly feeding HOCI solution to the substrate the substrate comprises a constant amount of aqueous HOCI solution. This benefits a constant and homogeneous evaporization of HOCI.

Alternatively or additionally, the HOCI solution from the container may be fed to the substrate at a bottom end of the substrate. In this case, capillary effects of the material of the substrate can transport the HOCI solution upwards into the substrate so that the HOCI solution is absorbed or adsorbed or received by the substrate.

In addition or as an alternative to feeding HOCI solution from the container to a top end and/or to a bottom end of the substrate, it may also be preferred that the fluid guiding means is provided and arranged to feed HOCI solution from the container to another contact area of the substrate.

Furthermore, preferably the substrate, and preferably the container and the fan, are arranged inside the housing, wherein the housing further comprises an inlet opening for ambient air and an outlet opening for the airflow transporting the HOCI evaporated from the substrate.

By arranging the substrate, and preferably the container and the fan, inside the housing stable and controllable conditions can be prepared in the housing to optimize the evaporation rate of the HOCI.

Still further preferably a filter, in particular a HEPA filter or a particulate filter, is arranged in the inlet opening, and/or a filter, in particular a HEPA filter or an aerosol separation filter, is arranged in the outlet opening.

By arranging a filter, in particular a HEPA-filter or a particulate filter, in the inlet opening contamination of the substrate from external contaminants can be prevented. By arranging a HEPA-filter or aerosol separation filter in the outlet opening any aerosol particles that potentially might be carried with the airflow carrying the evaporated HOCI can be filtered out and, thus, do not enter the ambient air.

Further preferably the apparatus further comprises a sensor for determining a concentration of HOCI in the ambient air, wherein the sensor is preferably arranged outside the housing.

By arranging the sensor outside of the housing, the HOCl concentration in the ambient air, in particular in the room air, can be measured in order to control the rate of HOCI being dispensed from the apparatus.

Preferably more than one sensor is arranged outside of the housing.

In a preferred embodiment the apparatus comprises a control unit for controlling a fan speed and/or for controlling a feed rate of the HOCI solution to the substrate, and/or for controlling a pressure and/or a temperature inside the housing for increasing the partial vapor pressure of HOCI compared to the partial vapor pressure of water.

By controlling the fan speed and/or controlling the feed rate of the HOCI solution to the substrate the rate of evaporation of HOCI and/or the amount of HOCI dispenses from the apparatus can be precisely controlled. In this case it is preferred that the control unit receives data from the sensor relating to the measured HOCI concentration in the ambient air. Thus, in the preferred embodiment a feedback loop between the measured HOCI concentration in the ambient air and the fan speed or the feed rate is provided via the control unit in order to adjust a stable concentration of HOCI in the ambient air.

Accordingly, the control unit is preferably configured for controlling the fan speed and/or the feed rate for stabilizing the HOCI concentration in the ambient air at a predetermined level.

In addition, the control unit or a second control unit may be configured for controlling a pressure and/or a temperature and/or a humidity inside the housing.

By controlling the pressure and/or the temperature and/or the humidity in the housing the partial vapor pressure of HOCI can be increased compared to the partial vapor pressure of water and thus the evaporation rate of HOCI can be further controlled.

For controlling the pressure and/or the temperature inside the housing the apparatus may further comprise a pressure generator and/or a heating element.

Still further preferably the apparatus comprises an electrolysis unit for producing the HOCI solution.

By providing an electrolysis unit for producing the HOCI solution a self-contain apparatus can be provided.

Preferably the electrolysis unit is configured for producing the HOCI solution with an HOCI concentration of 3000 ppm to 7000 ppm, further preferably of 4000 ppm to 6000 ppm, particularly preferably of 5000 ppm, and/or with a pH between 4 and 7, preferably with a pH between 5 and 6, still further preferably with a pH of 5.5, and/or with less than 10 gr./liter of salt, preferably with less than 8 gr./liter of salt, further preferably with less than 7 gr./liter of salt, particularly preferably with less than 6 gr./liter of salt.

It is also preferred that the apparatus comprises an excess fluid collecting means for collecting excess HOCI solution not being received by the substrate or not having wetted the substrate, wherein the excess fluid collecting means is preferably configured for returning the excess HOCI solution to the container.

When the substrate receives the HOCI solution or is wetted by the HOCI solution either via feeding the HOCI solution to the top of the substrate or to the bottom of the substrate, not all of the HOCI solution will be absorbed or adsorbed or received by the substrate. Thus, the excess fluid which has not been received or has not wetted the substrate is collected and returned to the HOCI container.

Furthermore, it may be preferred that the apparatus comprises a depleted fluid collecting means for collecting depleted HOCI solution after evaporation of HOCI from the HOCI solution received by the substrate, wherein the excess fluid collecting means is preferably configured for feeding the depleted HOCI solution to a depleted solution container.

Due to the evaporation of HOCI from the HOCI solution absorbed or adsorbed or received by the substrate, the HOCI concentration in the HOCI solution becomes depleted. The depleted HOCI solution will constantly flow down the substrate by gravity and is preferably collected at the bottom of the substrate. The depleted HOCI solution may not contain enough HOCl for further evaporation and, thus, it is preferred that the depleted HOCl solution is fed to a depleted solution container.

The object of the present invention is further solved by providing a sensor, preferably for an apparatus described above, wherein the sensor is an electrochemical sensor and wherein the electrochemical sensor has a cross sensitivity to free active chloride.

With the sensor of the invention its cross sensitivity to free active chloride can be used to determine the HOCl concentration in the ambient air.

An electrochemical sensor consists of at least two electrodes, a measuring and a counter electrode, which have contact which each other in two different ways: On the one hand via an electrically conductive medium, such as an electrolyte, on the other hand via an external electrical circuit. The electrodes have a catalytic effect so that a chemical reaction takes place at the so-called free phase boundary, where gas, catalyst and an electrolyte are present.

Preferably, the sensor comprises at least two electrodes, further preferably three electrodes, and an electrolyte layer.

A two-electrode sensor with a measuring and a counter electrode has the problem that higher gas concentrations lead to higher currents in the sensor and to a voltage drop. The voltage drop in turn changes the sensor voltage. This can lead to unstable measurement signals. Therefore, it is preferred that the sensor comprises a third electrode as a reference electrode, which does not carry current and whose potential remains constant. This leads to a considerably increased quality with regard to the linearity and selectivity of the sensor.

Another solution to the object of the invention is provided with a system for adjusting a stable concentration of hypochlorous acid and/or associated free active chlorine in ambient air for disinfection comprising an apparatus as described above.

Preferably the system comprises a sensor, in particular a sensor as described above, for determining a concentration of HOCl in the ambient air, and/or a control unit for controlling a fan speed and/or for controlling a feed rate of the HOCl solution to the substrate of the apparatus and/or for controlling a pressure and/or a temperature and/or a humidity inside the housing for increasing the partial vapor pressure of HOCI compared to the partial vapor pressure of water.

Still further preferably the system comprises a room air system and/or an HVAC system, wherein the apparatus and the room air system and/or the HVAC system are configured such that the room air system and/or the HVAC system receives an airflow transporting HOCl evaporated from the substrate.

Thus, the HOCl evaporated from the substrate is not directly dispensed into the ambient air from the apparatus, but instead is delivered to a room air system or to an HVAC system. The room air system or the HVAC system receives the airflow transporting the HOCI evaporated from the substrate and transports the HOCI throughout the rooms of a building.

A still further solution to the object of the invention is a method for adjusting a stable concentration of hypochlorous acid and/or associated free active chlorine in ambient air for disinfection, preferably conducted with a system as described above, wherein a substrate with a high surface-area-to-volume ratio is wetted by or receives an aqueous HOCI solution, wherein HOCI evaporates from the substrate, and wherein an airflow is generated preferably by a fan, and wherein HOCI evaporated from the substrate is transported to the ambient air.

In a preferred embodiment a fan speed of the fan and/or a feed rate of the HOCI solution to the substrate, and/or a temperature and/or pressure and/or a humidity is controlled to increase the partial vapor pressure of HOCI compared to the partial vapor pressure of water.

### Brief description of the figures

The invention is described with reference to the figures.
- Fig. 1: shows an apparatus for evaporating hypochlorous acid,
- Fig. 2: shows a sensor for the apparatus,
- Fig. 3: shows a system for adjusting a stable concentration of hypochlorous acid and/or associated free active chlorine in ambient air comprising the apparatus, and
- Fig. 4: shows a flow chart of a method for adjusting a stable concentration of hypochlorous acid and/or associated free active chlorine in ambient air.

### Detailed description of the figures

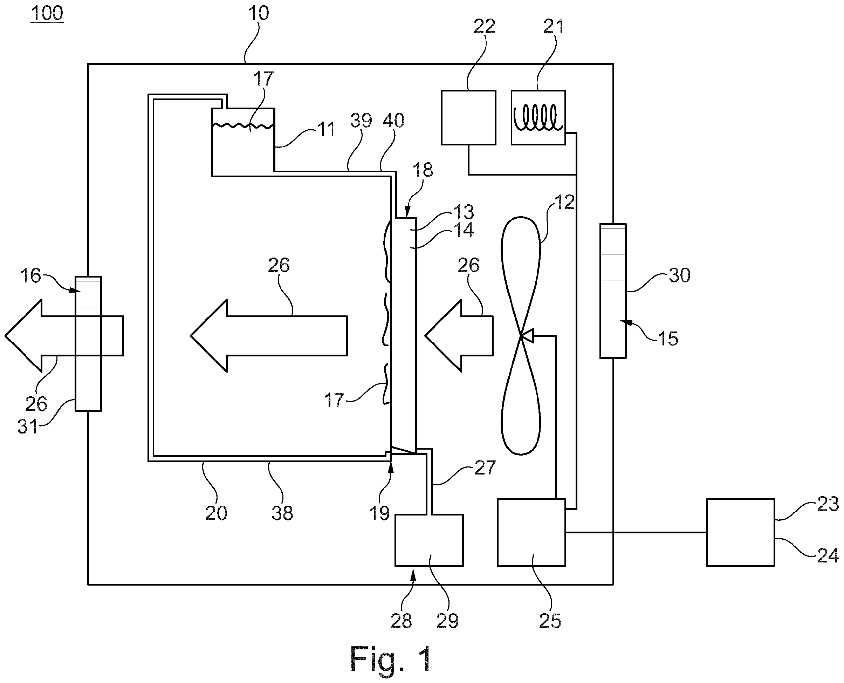

Fig. 1 shows an apparatus 100 for evaporating hypochlorous acid (HOCI) comprising a housing 10, a container 11 for an aqueous HOCI solution and a fan 12.

Furthermore, the apparatus 100 comprises a substrate 13 with a high surface-area-to-volume ratio. The substrate 13 is a mesh 14 which is resistant to HOCI. The substrate 13, the container 11 and the fan 12 are disposed inside the housing 10. The housing 10 furthermore comprises an inlet opening 15 for ambient air and an outlet opening 16.

In operation, HOCI solution 17 is fed to the top end 18 of the substrate 13 via line 39 of fluid guiding means 40 and flows down the substrate 13 due to gravity. Because of the high surface-area-to-volume ratio the substrate 13 absorbs or adsorbs or receives the HOCI solution 17. During operation the substrate 13 is constantly wetted. Excess HOCI solution 17 not being absorbed or adsorbed or received by the substrate 13 is collected at the bottom end 19 of the substrate 13 and is returned via a line 20 of an excess fluid collecting means 38 to the container 11.

The apparatus 100 furthermore comprises a heating element 21 and a pressure generator 22 for controlling the temperature and pressure inside the housing 10.

Still further the apparatus 100 comprises a sensor 23 arranged outside of the housing 10 for measuring the HOCI concentration in the ambient air. The sensor 23 is an electrochemical sensor 24 and has a cross sensitivity to free active chloride. By measuring the free active chloride, the HOCI concentration in the ambient air can be determined.

A control unit 25 receives measurement data from the sensor 23 and is configured to control the fan speed of the fan 12.

In operation of the apparatus 100, HOCI evaporates from the substrate 13. The fan generates an airflow 26, which passes through the substrate 13 and transports the evaporated HOCI out of the outlet opening 16 into the ambient air. The substrate 13 thus is air-permeable.

The control unit 25, the sensor 23 and the fan 12 form a feedback loop. The control unit 25 receives data from the sensor 23 regarding the HOCI concentration in the ambient air. The control unit 25 then controls the fan speed of the fan 12, thereby controlling the amount of HOCI transported to the ambient air. In addition, the control unit 25 controls the heating element 21 and the pressure generator 22 to adjust pressure and temperature conditions inside the housing 10, thereby increasing or decreasing the partial vapor pressure of HOCI compared to water. This allows for setting conditions inside the housing 10 that favor (or disfavor) the evaporation of HOCI over water from the substrate 13.

Depleted HOCI solution pools at the bottom end 19 of the substrate 13 and is transported away via a fluid line 27 of a depleted fluid collecting means 28. The fluid collecting means 28 comprises a depleted solution container 29 for the depleted HOCI solution.

A particulate filter 30 is arranged in the inlet opening 15 to prevent contamination of the substrate 13 from external contaminants. In the outlet opening 16 a HEPA filter 31 or aerosol separation filter is arranged. The HEPA-filter 31 or aerosol separation filter in the outlet opening 16 filters any aerosol particles that potentially might be carried with the airflow 26 and, thus, do not enter the ambient air.

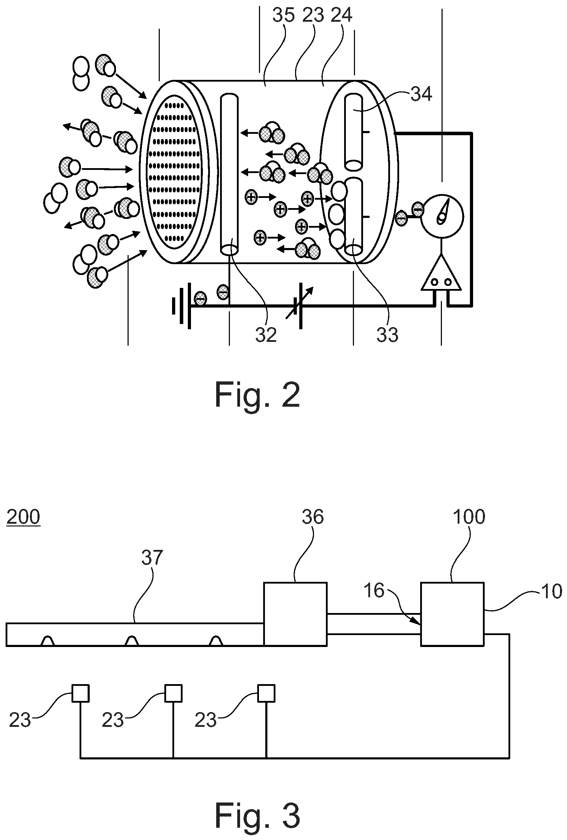

Fig. 2 shows a sensor 23 for the apparatus 100 according to fig. 1. The sensor 23 is configured as an electrochemical sensor 24 and comprises a measuring electrode 32, a counter electrode 33 and a reference electrode 34. The electrochemical sensor 24 further comprises an electrolyte layer 35.

Fig. 3 shows a system 200 for adjusting a stable concentration of hypochlorous acid and/or associated free active chlorine in ambient air for disinfection. The system 200 comprises an apparatus 100 of fig. 1 and multiple sensors 23 according to fig. 2. The system 200 furthermore comprises a room air system or an HVAC system 36. HOCI evaporated from the substrate 13 of the apparatus 100 is transported via the airflow 26 generated by the fan 12 out of the outlet opening 16 of the housing 10 of the apparatus 100 and into the HVAC system 36. The HVAC system 36 than distributes the HOCI evaporated from the substrate 13 to the ambient air via air delivery tubes 37. The sensors 23 measure the HOCI concentration in the ambient air and provide the measurement data to the control unit 25 of the apparatus 100 to control the fan speed of the fan 12.

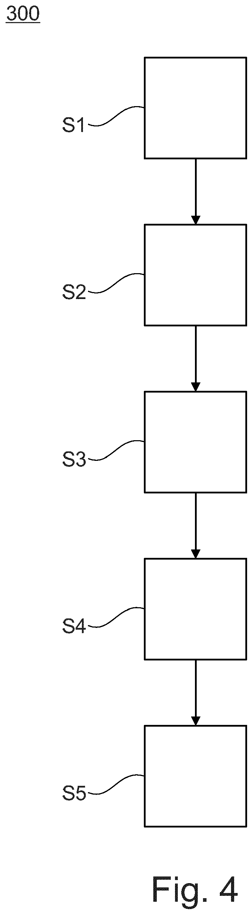

Fig. 4 shows a flow diagram of a method 300 for adjusting a stable concentration of hypochlorous acid and/or associated free active chlorine in ambient air for disinfection.

In a first step S1 a substrate 13 having a high surface-area-to-volume ratio is wetted by or receives and HOCI solution 17.

In a second step S2 HOCI evaporates from the substrate 13.

In a third step S3 an airflow generated by a fan 12 transports the evaporated HOCI to the ambient air.

In a fourth step S4 a sensor 23 determines the HOCI concentration in the ambient air and provides corresponding measurement data to a control unit 25. The control unit 25 then, in a fifth step, continuously compares the measured concentration of HOCI in the ambient air with a predetermined value and, if the measured concentration of HOCI in the ambient air differs from the predetermined value by a predetermined threshold, controls the speed of the fan 12 and/or the feed rate of the HOCI solution to the substrate 13 to adjust the concentration of the hypochlorous acid and/or associated free active chlorine in the ambient air to a stable value.

### List of reference numerals

- 100: Apparatus
- 200: System
- 300: Method
- 10: Housing
- 11: Container for an aqueous HOCI solution
- 12: Fan
- 13: Substrate
- 14: Mesh
- 15: Inlet opening
- 16: Outlet opening
- 17: HOCI solution
- 18: Top end
- 19: Bottom end
- 20: Fluid line
- 21: Heating element
- 22: Pressure generator
- 23: Sensor
- 24: Electrochemical sensor
- 25: Control unit
- 26: Airflow
- 27: Fluid line
- 28: Depleted fluid collecting means
- 29: Depleted solution container
- 30: Particulate filter
- 31: HEPA filter
- 32: Measuring electrode
- 33: Counter electrode
- 34: Reference electrode
- 35: Electrolyte layer
- 36: HVAC system
- 37: Air delivery tubes
- 38: Excess fluid collecting means
- 39: Line
- 40: Fluid guiding means

## Claims

1. Apparatus (100) for evaporating hypochlorous acid (HOCI), comprising a housing (10), a container (11) for an aqueous HOCI solution (17), and preferably a fan (12), **characterized in that** the apparatus further comprises a substrate (13) with a high surface-to-volume ratio arranged to receive or to be wetted by an HOCI solution (17) from the container (11) during operation of the apparatus (100).

2. Apparatus (100) according to claim 1, **characterized in that** the substrate (13) is a woven or non-woven fabric or a mesh (14) or open foam material, wherein the woven or non-woven fabric or mesh (14) or open foam material is preferably resistant to HOCI.

3. Apparatus (100) according to claim 1 or 2, **characterized in that** the fan (12) is arranged and configured to generate an airflow (26) for transporting HOCI evaporated from the aqueous HOCI solution (17) out of the apparatus, wherein said aqueous HOCI solution (17) has been received by or has wetted the substrate (13), wherein preferably the substrate (13) and fan (12) are arranged such that the airflow (26) generated by the fan (12) passes through or along the substrate (13).

4. Apparatus (100) according to one of the preceding claims, **characterized in that** the substrate (13) receives or is wetted by the HOCI solution (17) by the HOCI solution (17) running down the substrate (13) following gravity or by the HOCI solution (17) being absorbed or adsorbed by a capillary effect, and/or that a fluid guiding means (40) is provided and arranged to feed HOCI solution (17) from the container (11) to a top end (18) and/or to a bottom end (19) of the substrate (13).

5. Apparatus (100) according to one of the preceding claims, **characterized in that** the substrate (13), and preferably the container (11) and the fan (12), are arranged inside the housing (10), wherein the housing (10) further comprises an inlet opening (15) for ambient air and an outlet opening (16) for the airflow (26) transporting the HOCI evaporated from the substrate (13), wherein preferably a filter, further preferably a HEPA filter or a particulate filter (30), is arranged in the inlet opening (15), and/or wherein preferably a filter, further preferably a HEPA filter (31) or an aerosol separation filter, is arranged in the outlet opening (16).

6. Apparatus (100) according to one of the preceding claims, **characterized in that** the apparatus further comprises a sensor (23) for determining a concentration of HOCI in the ambient air, wherein the sensor (23) is preferably arranged outside the housing (10).

7. Apparatus (100) according to one of the preceding claims, **characterized in that** the apparatus comprises a control unit (25) for controlling a fan speed and/or for controlling a feed rate of the HOCI solution (17) to the substrate (13), and/or for controlling a pressure and/or a temperature and/or a humidity inside the housing (10) for increasing the partial vapor pressure of HOCI compared to the partial vapor pressure of water.

8. Apparatus (100) according to one of the preceding claims, **characterized in that** the apparatus comprises an electrolysis unit for producing the HOCI solution (17).

9. Apparatus (100) according to one of the preceding claims, **characterized in that** the apparatus comprises an excess fluid collecting means (38) for collecting excess HOCI solution (17) not being received by the substrate (13) or not having wetted the substrate (13), wherein the excess fluid collecting means (38) is preferably configured for returning the excess HOCI solution (17) to the container (11), and/or that the apparatus comprises a depleted fluid collecting means (28) for collecting depleted HOCI solution (17) after evaporation of HOCI from the HOCI solution (17) received by the substrate (13), wherein the depleted fluid collecting means (28) is preferably configured for feeding the depleted HOCI solution (17) to a depleted solution container (29).

10. Sensor (23) for an apparatus (100) according to one of claims 6 to 9, wherein the sensor (23) is an electrochemical sensor (24), **characterized in that** the electrochemical sensor (24) has a cross-sensitivity to free active chlorite.

11. Sensor (23) according to claim 10, **characterized in that** the sensor (23) comprises at least two electrodes (32, 33), preferably three electrodes (32, 33, 34), and an electrolyte layer (35).

12. System (200) for adjusting a stable concentration of hypochlorous acid and/or associated free active chlorine in ambient air for disinfection comprising an apparatus (100) according to one of claims 1 to 9.

13. System (200) according to claim 12 **characterized in that** the system comprises a sensor (23), preferably according to claims 10 or 11, for determining a concentration of HOCI in the ambient air, and/or a control unit (25) for controlling a fan speed and/or for controlling a feed rate of the HOCI solution (17) to the substrate (13) of the apparatus (100) and/or for controlling a pressure and/or a temperature and/or a humidity inside the housing (10) for increasing the partial vapor pressure of HOCI compared to the partial vapor pressure of water.

14. System (200) according to claim 12 or 13 **characterized in that** the system (200) comprises a room air system and/or an HVAC system (36), wherein the apparatus (100) and the room air system and/or the HVAC system (36) are configured such that the room air system and/or the HVAC system (36) receives an airflow (26) transporting HOCl evaporated from the substrate (13).

15. Method (300) for adjusting a stable concentration of hypochlorous acid and/or associated free active chlorine in ambient air for disinfection, preferably conducted with a system (200) according to any one of claims 12 to 14, wherein a substrate (13) with a high surface-to-volume ratio is wetted by or receives an aqueous HOCI solution (17), wherein HOCI evaporates from the substrate (13), and wherein an airflow (26) is generated preferably by a fan (12), and wherein HOCI evaporated from the substrate (13) is transported to the ambient air.

16. Method (300) according to claim 15, **characterized in that** a fan speed of the fan (12) and/or a feed rate of the HOCI solution (17) to the substrate (13), and/or a temperature and/or pressure and/or a humidity is controlled to increase the partial vapor pressure of HOCI compared to the partial vapor pressure of water.
